(19) Europäisches Patentamt
European Patent Office
Office européen des brevets

(11) EP 1 151 988 A1

(12) EUROPÄISCHE PATENTANMELDUNG

(43) Veröffentlichungstag:
**07.11.2001 Patentblatt 2001/45**

(51) Int Cl.[7]: **C07C 45/00**, C07C 47/04, B01J 23/04

(21) Anmeldenummer: **01118017.1**

(22) Anmeldetag: **26.05.1998**

(84) Benannte Vertragsstaaten:
**BE DE FR GB IT NL**

(30) Priorität: **02.06.1997 DE 19722774**
**30.06.1997 DE 19727519**
**30.06.1997 DE 19727520**
**30.09.1997 DE 19743145**
**31.03.1998 DE 19814283**

(62) Dokumentnummer(n) der früheren Anmeldung(en) nach Art. 76 EPÜ:
**98924318.3 / 0 986 528**

(71) Anmelder: **Ticona GmbH**
**65451 Kelsterbach (DE)**

(72) Erfinder:
- **Shweers, Elke, Dr.**
  **65812 Bad Soden (DE)**
- **Kaiser, Thomas, Dr.**
  **65779 Kelkheim (DE)**
- **Haubs, Michael, Dr.**
  **55545 Bad Kreuznach (DE)**
- **Rosenberg, Michael**
  **65527 Niedernhausen (DE)**

Bemerkungen:
Diese Anmeldung ist am 25.07.2001 als Teilanmeldung zu der unter INID-Kode 62 erwähnten Anmeldung eingereicht worden.

(54) **Verfahren zur nicht oxidativen Herstellung von Formaldehyd aus Methanol**

(57) Verfahren zur Herstellung von Formaldehyd aus Methanol durch Dehydrierung des Methanols bei einer Temperatur im Bereich von 300 bis 1000°C in Gegenwart einer aus einer Natriumverbindung freigesetzten katalytisch wirksamen Spezies, dadurch gekennzeichnet, daß man aus einer Natriumverbindung aus der Gruppe

e) Natriumalkoholate
f) Natriumcarboxylate
g) Natriumsalze C-H acider Verbindungen
h) Natriumoxid, Natriumhydroxid, Natriumnitrit, Natriumacetylid, Natriumcarbid, Natriumhydrid und Natriumcarbonyl

durch thermische Zersetzung die katalytisch aktive Spezies freisetzt.

Fig. 1

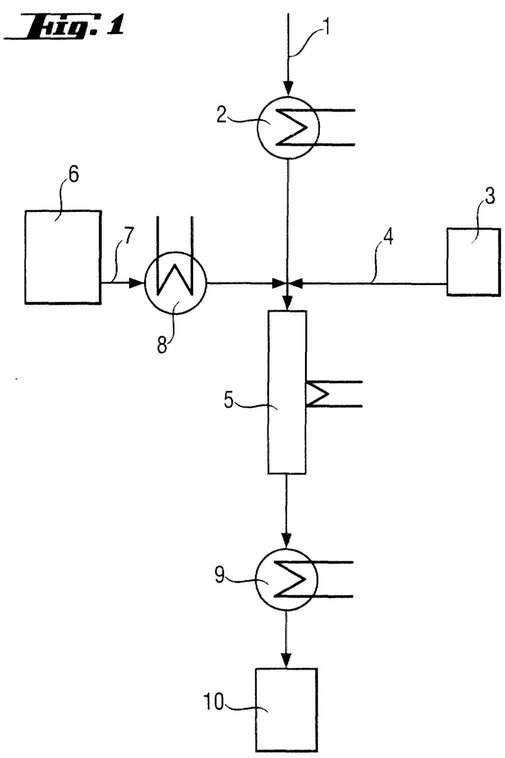


EP 1 151 988 A1

## Beschreibung

Mehrere Verfahren zur Herstellung von Formaldehyd aus Methanol sind bekannt (siehe z.B. Ullmann's Encyclopaedia of Industrial Chemistry). Technisch durchgeführt werden überwiegend die Oxidation

$$CH_3OH + \tfrac{1}{2}\, O_2 \rightarrow CH_2O + H_2O$$

an Eisen- und Molybdänoxyd enthaltenden Katalysatoren bei 300°C bis 450°C (Formox Prozeß) und die oxidative Dehydrierung (Silberkontakt - Verfahren) gemäß:

$$CH_3OH \rightarrow CH_2O + H_2$$

$$H_2 + \tfrac{1}{2}\, O_2 \rightarrow H_2O$$

bei 600°C bis 720°C. Nach beiden Verfahren liegt der Formaldehyd zunächst als wäßrige Lösung vor. Insbesondere bei der Verwendung für die Herstellung von Formaldehyd - Polymeren und -Oligomeren muß der so gewonnene Formaldehyd aufwendig entwässert werden. Ein weiterer Nachteil ist die Bildung korrosiver, die Polymerisation negativ beeinflussender Ameisensäure als Nebenprodukt.

Durch die Dehydrierung von Methanol können diese Nachteile vermieden und kann im Gegensatz zu oben genannten Verfahren nahezu wasserfreier Formaldehyd direkt gewonnen werden:

$$CH_3OH \xrightarrow{\text{Kat.}} CH_2O + H_2$$

Um ein ökologisches und wirtschaftlich interessantes technisches Verfahren für die Dehydrierung von Methanol zu erreichen, sollten die folgenden Voraussetzungen erfüllt werden: Die stark endotherme Reaktion sollte bei hohen Temperaturen durchgeführt werden, damit hohe Umsätze erreicht werden. Konkurrierende Nebenreaktionen müssen unterdrückt werden, um eine hinreichende Selektivität für Formaldehyd zu erzielen (unkatalysiert beträgt die Selektivität für die Bildung von Formaldehyd bei Umsätzen über 90 % weniger als 10 %). Die Verweilzeiten müssen kurz oder die Abkühlung der Reaktionsprodukte schnell sein, um den Zerfall des thermodynamisch bei den Reaktionsbedingungen nicht stabilen Formaldehyds

$$CH_2O \rightarrow CO + H_2$$

zu verringern.

Verschiedene Verfahren zur Durchführung dieser Reaktion wurden vorgeschlagen; so ist beispielsweise in der DE-A-37 19 055 ein Verfahren zur Herstellung von Formaldehyd aus Methanol durch Dehydrierung in Gegenwart eines Katalysators bei erhöhter Temperatur beschrieben. Die Umsetzung wird in Gegenwart eines mindestens eine Natriumverbindung enthaltenden Katalysators bei einer Temperatur von 300°C bis 800°C durchgeführt.

J. Sauer und G. Emig (Chem. Eng. Technol. 1995, 18, 284-291) gelang es, aus einem $NaAlO_2$ und $LiAlO_2$ enthaltenden Katalysator durch ein reduzierendes Gasgemisch (87%$N_2$ + 13%$H_2$) eine katalytisch aktive Spezies freizusetzen, bei der es sich seiner Vermutung nach um Natrium handelt. Diese Spezies vermag die Dehydrierung von im selben Reaktor stromabwärts zugegebenem, d.h. nicht mit der Katalysatorschüttung in Kontakt gekommenem, Methanol zu Formaldehyd zu katalysieren. Bei Verwendung nicht reduzierender Gase wurde nur geringe katalytische Aktivität beobachtet.

Nach J. Sauer und G. Emig sowie Ergebnissen aus neueren Untersuchungen (siehe z.B. M. Bender et al., Vortrag auf dem XXX. Jahrestreffen deutscher Katalytiker, 21.-23.3.1997) wurden Natriumatome und NaO-Moleküle als in die Gasphase emittierte Verbindungen identifiziert und deren katalytische Aktivität für die Dehydrierung von Methanol in der Gasphase beschrieben.

Das Ausgangsmaterial Methanol wird bei den bekannten Verfahren stets verdünnt mit Stickstoff und/oder Stickstoff/ Wasserstorf-Gemischen umgesetzt.

Obwohl mit den bekannten Verfahren bereits gute Ergebnisse erzielt werden, besteht doch ein breiter Raum

für Verbesserungen in technischer und ökonomischer Hinsicht, besonders weil sich die eingesetzten Katalysatoren mit der Zeit verbrauchen oder inaktivieren sowie die Formaldehydausbeuten noch verbesserungsfähig sind.

Es wurde nun überraschend gefunden, daß die Ausbeute der Dehydrierung gesteigert werden kann, wenn ein Trägergasstrom, der durch Erwärmung auf eine Temperatur über der eigentlichen Reaktionstemperatur gebracht wurde, in den Reaktor eingeleitet wird. Durch einen solchen überhitzten Trägergasstrom kann zumindest ein Teil der für die endotherme Dehydrierungsreaktion benötigten Wärmemenge eingebracht werden.

Ein Vorteil hierbei ist, daß die Reaktionswärme nicht über eine heiße, d.h. eine Temperatur über der Reaktionstemperatur aufweisende, Wand in der Reaktionszone auf den Gasstrom übertragen werden muß, sondern durch die getrennte Temperierung und intensive Mischung der verschiedenen Teilströme direkt und schonender für die Reaktionsgase eingebracht werden kann. Eine Zersetzung des instabilen Formaldehyds sowie Nebenreaktionen bei den hohen Temperaturen im Reaktor, insbesondere in den wandnahen Zonen, können so vermindert werden.

Gegenstand der Erfindung ist daher ein Verfahren zur Herstellung von Formaldehyd aus Methanol durch Dehydrierung in einem Reaktor in Gegenwart eines Katalysators bei einer Temperatur im Bereich von 300 bis 1000°C, dadurch gekennzeichnet, daß dem Reaktor ein Trägergasstrom zugeführt wird, der eine Temperatur aufweist, die oberhalb der Dehydrierungstemperatur liegt.

Bevorzugt beträgt die Temperaturdifferenz zwischen Trägergasstrom und Dehydrierungstemperatur mindestens 20°C, besonders bevorzugt 40 bis 250°C.

Der überhitzte Gasstrom kann direkt in die Reaktionszone geführt werden oder ganz oder teilweise vorher mit einem Primärkatalysator (s.u.) in Kontakt gebracht werden.

Für den überhitzten Gasstrom liegen die bevorzugten Temperaturen zwischen 600 und 1000 °C, besonders bevorzugt zwischen 700 und 900 °C. Bevorzugte Temperaturen zur Dehydrierung des Methanols liegen zwischen 500 und 900°C, besonders bevorzugt sind Temperaturen zwischen 600 und 800°C.

Der oder die Trägergasströme können aus einem reduzierenden oder nicht reduzierendem Gas, wie $H_2$/CO -Gemischen oder Stickstoff, vorzugsweise aus den Nebenprodukten der Dehydrierung, bestehen.

Figur 1 gibt einen schematischen Überblick über eine bevorzugte Variante des erfindungsgemäßen Verfahrens.

Der Trägergasstrom 1 wird im Wärmeaustauscher 2 erwärmt. Zusammen mit dem aus einem Vorratsgefäß 3 kommenden Katalysator 4 wird der Gesamtstrom in den Reaktor 5 geleitet. Aus einem Vorratsgefäß 6 wird Methanol 7 gefördert, in einem Wärmeaustauscher 8 verdampft, erwärmt und ebenfalls in den Reaktor 5 geführt. Die Produktgase aus dem Reaktor 5 werden im Wärmeaustauscher 9 abgekühlt und einer Einheit 10 zum Abtrennen des Formaldehyds zugeführt.

Weiterhin Gegenstand der Erfindung ist eine Vorrichtung zur Durchführung des obengenannten Verfahrens enthaltend einen oder mehrere Wärmeaustauscher zum Vorwärmen der Ausgangsstoffe, einen Behälter zur Überhitzung eines Trägergasstromes, einen beheizten Reaktor zur Durchführung der Dehydrierung, einen oder mehrere Wärmeaustauscher zum Abkühlen des Produktgemisches, eine Einrichtung zum Abtrennen des Formaldehyds sowie eine Vorrichtung zum Einbringen des Methanols und zur Nachführung eines Katalysators.

Dehydrierung bedeutet im Sinne der Erfindung einen nicht-oxidativen Prozeß gemäß der Gleichung:

$$CH_3OH \xrightarrow{\text{Kat.}} CH_2O + H_2$$

Geeignete Katalysatoren sind beispielsweise aus der Literatur bekannt, siehe z.B. Chem. Eng. Technol. 1994, 17, 34.

An Metallen eignen sich beispielsweise Li, Na, K, Cs, Mg, Al, In, Ga, Ag, Cu, Zn, Fe, Ni, Co, Mo, Ti, Pt, bzw. deren Verbindungen. Weiterhin geeignet sind beispielsweise S, Se, Phosphate von Übergangsmetallen, wie V und Fe, und Heteropolysäuren, wie Molybdatophosphorsäure.

Beispiele für konkrete Katalysatoren sind:

- Natrium oder Natriumverbindungen (DE-A-37 19 055 und DE-A-38 11 509)
- Aluminiumoxid, Alkalialuminat und/oder Erdalkalialuminat (EP-A-04 05 348)
- Silberoxid (JP-A 60/089 441, Dervent Report 85 - 15 68 91/26)
- ein Kupfer, Zink und Schwefel enthaltender Katalysator (DE-A 25 25 174)
- ein Kupfer, Zink und Selen enthaltender Katalysator (US-A 4,054,609)
- ein Zink und/oder Indium enthaltender Katalysator (EP-A 0 130 068)
- Silber (US-A 2,953,602)
- Silber, Kupfer und Silicium (US-A 2,939,883).

- Zink, Cadmium, Selen, Tellur oder Indium enthaltende Verbindungen.

Bevorzugt ist die Verwendung von Natrium oder Natriumverbindungen.

Die Anwendungsform eines solchen, beispielsweise natriumhaltigen Katalysators kann in weitem Rahmen variieren:
Metallisch, z.B. auch als Legierung mit mindestens einem anderen Legierungsbestandteil, als Verbindung oder Salz, wobei mindestens ein nichtmetallisches Element mit Na gebunden vorliegt (binäre Verbindungen und Salze). Ist mehr als ein Element in der Verbindung gebunden, so liegen binäre, ternäre oder quartemäre Verbindungen und Salze vor. Ebenso bevorzugt ist der Einsatz in geträgerter Form, beispielsweise auf einem anorganischen Träger.

Wird Natrium metallisch eingesetzt, so kann es fest, flüssig oder bevorzugt dampfförmig eingesetzt werden. Bevorzugte Legierungen sind solche mit anderen Alkalimetallen und/oder Erdalkalimetallen, wie Ba, Sr, Ca, Cs, Rb, K oder besonders bevorzugt Li und/oder Magnesium.

Darüber hinaus können auch Legierungen mit B, Al, Si und Sn Verwendung finden. Dies gilt besonders auch für Legierungen, die Verbindungen wie Natriumborid, $NaB_2$, Natriumsilicid, NaSi, oder NaSn enthalten können.

Geeignete binäre Na-Verbindungen und Salze sind beispielsweise Natriumcarbide, wie $Na_2C_2$, $NaC_8$, Natriumhalogenide, wie NaF, Natriumoxide, wie $Na_2O$, Natriumazid, Natriumphosphid, Natriumsulfid, Natriumpolysulfide, bevorzugt auch Natriumhydride, wie NaH.

Geeignete temäre Na-Verbindungen und Salze sind beispielsweise Natriumborate, wie Borax, Natriumphosphate bzw. -hydrogenphosphate, Natriumphosphite, Natrium(meta)silikate und -alumosilikate, wie Wasserglas, $Na_3AlF_6$ (Kryolith), Natrium(hydrogen)sulfat, Natriumsulfit, Natriumnitrit, Natriumnitrat, Natriumamid, Natriumacetylid NaCCH, Natriumcyanid, Natriumrhodanid, Natriumthiomethylat, Natriumthiosulfat, bevorzugt jedoch NaOR, mit R = H, organischer Rest (= Salze organischer Säuren, Alkoholate, Phenolate, Acetylacetonat, Acetessigsäureestersalz, Salze der Salicylsäure oder des Salicylaldehyds), Natriumcarbonat und Natriumhydrogencarbonat und deren Gemische, wie Soda, Thermonatrit, Trona, Pirssonit, Natrocalcit. Generell ist der Einsatz wasserfreier, d.h. getrockneter Salze vorzuziehen.
Besonders bevorzugt sind NaOH, NaOOC-R' (vorzugsweise Formiat, Acetat, Lactat, Oxalat), NaOR' (R' ist ein organisches Radikal mit 1 bis 4 C-Atomen) und Natriumcarbide.
Ganz besonders bevorzugt sind NaOH, Natriumformiat, Natriummethylat, Natriumacetat und Natriumcarbide, wie $Na_2C_2$.

Geeignete quarternäre Verbindungen sind z.B. auch natriumhaltige Alumosilikate, die künstlich hergestellt werden können oder auch vielfältig als natürliche Mineralien und Gesteine (z.B. Natronfeldspat oder Albit und Kalk-Natronfeldspat oder Oligoklas) vorkommen. Sie können zusätzlich durch lonenaustausch mit Na beladen werden.

Vorteilhaft können auch Doppelsalze vom Typ des Alauns oder Thenardit, Glauberit, Astrakanit, Glaserit, Vanthoffit Verwendung finden.

Die hier genannten Natriumverbindungen und Salze können vorteilhaft auch als Gemische vorliegen. Insbesondere sind durchaus auch Gehalte von < 50%, bevorzugt < 30% von Kationen anderer Alkalimetalle und/oder Erdalkalimetalle, wie Ba, Sr, Ca, Cs, Rb, K oder bevorzugt Li und / oder Magnesium einsetzbar. Besonders vorteilhaft sind technisch erhältliche, komplexe Gemische, wie Natronkalk, Thomasmehl und Zemente, z.B. Portlandzement, gegebenenfalls nach Anreicherung mit Natrium durch Lagerung in natriumhaltigen Lösungen (NaCl, Meerwasser).

Insbesondere bevorzugt sind Natriumverbindungen aus der Gruppe:

a) Natriumalkoholate,
b) Natriumcarboxylate,
c) Natriumsalze C-H acider Verbindungen,
d) Natriumoxid, Natriumhydroxid, Natriumnitrit, Natriumacetylid, Natriumcarbid Natriumhydrid und Natriumcarbonyl.

Die oben genannten Katalysatoren werden im folgenden als Primärkatalysator bezeichnet.

Obengenannte Verbindungen liefern in dem erfindungsgemäßen Verfahren Formaldehyd-Ausbeuten von über 60 % und geringe Wasserkonzentrationen von weniger als 5 mol % $H_2O$ pro mol Formaldehyd auch bei Reaktionstemperaturen von 600 bis 1000 °C

Die Freisetzung der katalytisch aktiven Spezies aus dem Primärkatalysator erfolgt vorzugsweise durch dessen thermische Zersetzung.

Der Primärkatalysator kann beispielsweise als Feststoff, in einem Lösungsmittel gelöst, als Flüssigkeit oder als Schmelze jeweils kontinuierlich oder diskontinuierlich zu- oder nachgeführt werden.

Die Nachführung des Primärkatalysators als Feststoff, z.B. pulverförmig, körnig oder kompaktiert, erfolgt im allgemeinen über eine Feststoffdosierung, z.B. mit Hub- oder Drehkolben, Zellradschleuse, Schnecke oder Schüttelrinne.

Wird der Primärkatalysator gelöst zugegeben, sind besonders Lösungsmittel mit einer chemischen Zusammensetzung geeignet, die nur die im Prozeß schon vorhandenen Elemente (C,H,O) enthalten. Besonders bevorzugt ist MeOH als Lösungsmittel. Die Zugabe erfolgt z.B. über eine Düse, die gekühlt werden kann, um ein Verdampfen des Lösungsmittels, Auskristallisieren oder Ablagerungen des festen Primärkatalysators in der Düse zu vermeiden.

Die Zugabe des Primärkatalysators als Schmelze ist z.B. über eine Düse möglich. Die Schmelze kann dann direkt im Gasstrom verdampft oder zersetzt werden.

Bei allen Möglichkeiten der Primärkatalysatornachführung geschieht dies vorteilhaft in einer Art und Weise, daß das Material in intensivem Kontakt mit strömendem Gas steht. Dies kann beispielsweise durch Aufbringen des Katalysatormaterials nach den oben beschriebenen Verfahren auf eine geeignete Oberfläche erreicht werden, die vom Gas durch - oder überströmt werden. Es kann sich hierbei um die Oberfläche eines Trägermaterials handeln, das in einem Festbett als Schüttung angeordnet ist. Als Materialien eignen sich z.B. SiC, $SiO_2$ und $Al_2O_3$ in einer geeigneten geometrischen Form, z.B. als Granulat, Pellets oder Kugeln. Die Anordnung geschieht vorzugsweise senkrecht in einem Festbett, vorzugsweise mit Dosierung von oben. Die eingetragene Substanz schlägt sich auf dem Trägermaterial nieder und die katalytisch aktive Spezies geht während des Prozesses in die Gasphase über.

Eine andere Möglichkeit ist die Anordnung des Primärkatalysators in einer Wirbelschicht, durch die der Trägergasstrom geleitet wird. Das Wirbelgut besteht dabei zumindest teilweise aus dem geträgerten oder ungeträgerten Primärkatalysator. Der Verlust an aktiver Substanz kann durch Nachführung von frischem Primärkatalysator ersetzt werden, verbrauchtes Material kann gegebenenfalls abgezogen werden. Dies kann im kontinuierlichen Fall beispielsweise durch eine zirkulierende Wirbelschicht verwirklicht werden.

Eine Nachführung des Primärkatalysators kann auch durch abwechselnde Sekundärkatalysatorerzeugung in verschiedenen Behältern erfolgen, in denen der Primärkatalysator beispielsweise als Festbett oder Wirbelschicht, jeweils geträgert oder ungeträgert, angeordnet sein kann.
Der Vorteil einer Verwendung mehrerer Einheiten zur diskontinuierlichen Katalysatornachführung besteht darin, daß auch solche Primärkatalysatoren eingesetzt werden können, bei denen, z.B. aufgrund von Stoffeigenschaften, wie Schmelzpunkt, Viskosität oder Zersetzungstemperatur, eine kontinuierliche Förderung nicht oder nur mit großem Aufwand möglich wäre.

In einer bevorzugten Variante des erfindungsgemäßen Verfahrens erfolgt die Erzeugung des Sekundärkatalysators räumlich getrennt von der Reaktionszone, in der die eigentliche Dehydrierung stattfindet, und bei einer Temperatur oberhalb der Dehydrierungstemperatur.
Bevorzugt beträgt die Temperaturdifferenz zwischen dem Ort der Katalysatorerzeugung und der Reaktionszone mindestens 20°C, besonders bevorzugt 40 bis 250°C.

Aus den erfindungsgemäßen Primärkatalysatoren werden bei thermischer Behandlung in der Primärkatalysatorzersetzungszone und beim Überströmen mit einem reduzierenden oder auch mit einem nicht reduzierendem Gas, wie molekularem Stickstoff, bei Temperaturen, die nicht gleich der Reaktionstemperatur für die Dehydrierung sondern höher oder tiefer sein können, eine oder mehrere katalytisch aktive Spezies ausgetragen bzw. Erzeugt und/oder auf ihm erzeugt (Sekundärkatalysator), die in der Lage sind, die Dehydrierung von Methanol zu katalysieren. Ein solch fluider Katalysator kann über erhebliche Strecken transportiert werden, ohne einen erheblichen Verlust an Wirksamkeit in der Dehydrierung zu erleiden. Diese getrennte Temperatureinstellung erlaubt durch Anpassung an die jeweiligen Bedingungen zur Katalysatorfreisetzung/-verdampfung bzw. Erzeugung einer katalytisch aktiven Spezies (Sekundärkatalysator) einerseits und zur Reaktion andererseits insbesondere die Möglichkeit zur Erniedrigung der Reaktionstemperatur. Damit vermindert sich der Zerfall des unter Reaktionsbedingungen instabilen Formaldehyds durch Folgereaktionen und erhöht sich die Ausbeute.

Bevorzugte Temperaturen zur Erzeugung des Sekundärkatalysators aus dem Primärkatalysator liegen zwischen 300 und 1100°C, besonders bevorzugt sind Temperaturen zwischen 400 und 1000°C.

Weiterhin können die Verweilzeiten im Dehydrierungsreaktor und Behälter zur Primärkatalysatorzugabe bzw. zur Erzeugung des Sekundärkatalysators über die Aufteilung des Trägergasstromes getrennt eingestellt werden. Dadurch wird eine gezielte Beladung des durch die Katalysatorzugabeeinheit geleiteten Gasstromes mit der aktiven Spezies erreicht.

Bevorzugte Verweilzeiten zur Erzeugung des Sekundärkatalysators liegen zwischen 0,01 und 60 sec, besonders bevorzugt zwischen 0,05 und 3 sec.

Für die Reaktion kann handelsübliches Methanol eingesetzt werden, vorzugsweise sollte es wasserarm sein und keine Stoffe enthalten, die den Katalysator vergiften.

Zur Durchführung der Dehydrierung wird das fluide, vorzugsweise gasförmige Methanol vorzugsweise mit Trägergas verdünnt.

Der molare Methanolanteil beträgt im allgemeinen 5 bis 90 %, vorzugsweise 10 bis 50 %, besonders bevorzugt 10 bis 40 %.

Der Druck ist bei dem erfindungsgemäßen Verfahren unkritisch. Die Dehydrierung des Methanols kann bei Unterdruck, Normaldruck oder Überdruck durchgeführt werden. Ein Bereich von etwa 0,1 bis 10 bar, vorzugsweise 0,5

bis 2 bar, ist besonders geeignet. Bevorzugt wird Normaldruck. Das erfindungsgemäße Verfahren kann diskontinuierlich oder kontinuierlich durchgeführt werden, wobei letzteres bevorzugt ist. Die Temperatur beträgt im allgemeinen 300°C bis 950°C, bevorzugt 500 bis 900°C, besonders bevorzugt 600 bis 850°C.

Wird die Erzeugung des Sekundärkatalysators räumlich getrennt von der Reaktionszone durchgeführt, liegen die Temperaturen in der Reaktionszone im allgemeinen zwischen 200 und 1000°C, vorzugsweise zwischen 300°C und 980°C. Es werden vorzugsweise 0,01 bis 1 kg Methanol pro Stunde und pro Gramm eingesetzten Katalysator umgesetzt. Im Falle eines kontinuierlichen Prozesses muß der Katalysator kontinuierlich oder diskontinuierlich nachgeführt werden. Die Mengen betragen dabei im allgemeinen 10 Milligramm bis 5 Gramm, vorzugsweise 10 mg bis 1 g, besonders bevorzugt 50 bis 1000 mg, ganz besonders 50 mg bis 500 mg pro kg umgesetztes Methanol.

Zur Dehydrierung des Methanols sind Verweilzeiten in der Reaktionszone von 0,005 bis 30 sec bevorzugt, besonders bevorzugt sind 0,01 bis 15 sec, ganz besonders bevorzugt 0,05 bis 3 sec.

Geeignete Reaktoren sind dem Fachmann bekannt und geläufig. Grundsätzlich können Reaktortypen und Aufbauten verwendet werden, wie sie aus der Literatur für Dehydrierungsreaktionen bekannt sind. Solche Apparaturen sind beispielsweise in Winnacker/Küchler, Chemische Technologie, 4. Auflage, Kapitel "Technik der Pyrolyse" Hanser Verlag, München 1981-86, beschrieben.
Geeignet sind beispielsweise Rohrreaktoren; geeignete Reaktormaterialien sind beispielsweise keramische Werkstoffe, wie Korund, aber auch aufkohlungs-, temperatur- und zunderbeständige Eisen- und Nickelbasislegierungen, wie Inconel 600® oder Hasteloy® .

Wird der Reaktor 5 oder der Behälter 2 durch eine Verbrennungsreaktion beheizt, eignen sich z.B. von außen befeuerte Rohre.

Ebenfalls bevorzugt ist die Beheizung des Reaktors durch Mikrowellen.

In einer weiteren bevorzugten Variante des erfindungsgemäßen Verfahrens wird ein Kreisgasstrom, der im wesentlichen aus Nebenprodukten der Dehydrierung besteht, durch den Reaktor geleitet.

Weiterhin bevorzugt ist es, daß man einen Teil der Nebenprodukte aus dem Kreisgasprozeß ausschleust und zur Befeuerung des Reaktors verwendet.

Die Abtrennung des Formaldehyds aus dem Reaktionsgemisch kann nach an sich bekannten, dem Fachmann geläufigen Methoden erfolgen, beispielsweise durch Polymerisation, Kondensation oder physikalische oder chemische Ab- oder Adsorption.

Eine technisch erprobte Methode ist die Bildung von Halbacetalen aus Formaldehyd und einem Alkohol. Die Halbacetale werden daran anschließend thermisch gespalten, wobei sehr reiner Formaldehyd-Dampf entsteht. Als Alkohol wird meist Cyclohexanol verwendet, da dessen Siedepunkt genügend weit über der Zersetzungstemperatur des Halbacetals liegt. Die Halbacetale werden üblicherweise in Fallfilm- oder Dünnschichtverdampfern bei Temperaturen von 100 bis 160°C gespalten (siehe z.B. US 2,848,500 vom 19.08.1958 "Preparation of Purified Formaldehyde" und US 2,943,701 vom 05.07.1960 "Process for purification of gaseous formaldehyde", oder JP-A 62/289 540). Die dabei freiwerdenden Formaldehyd-Dämpfe enthalten noch geringe Mengen Verunreinigungen, die meist durch eine Gegenstromwäsche mit Alkohol, wie Cyclohexanolhemiformal, durch Kondensation oder auch durch gezielte Präpolymerisation, entfernt werden.

Besonders bevorzugte Methoden zur Reinigung des erfindungsgemäß hergestellten Formaldehyds sind in den deutschen Patentanmeldungen 19 747 647.3 und 19 748 380.1 beschrieben.

Eine weitere Methode zur Abtrennung von Formaldehyd aus dem Reaktionsgemisch ist die Bildung von Trioxan in einem katalytischen Gasphasenprozeß (siehe z.B. Appl. Catalysis A 1997, 150, 143-151 und EP-A 0 691 338). Trioxan kann dann z.B. auskondensiert werden.

Verwertungsmöglichkeiten für die Nebenprodukte der Reaktion, insbesondere Wasserstoff, sind beispielsweise die Synthese von Methanol oder die Gewinnung von reinem Wasserstoff, der z.B. durch Membranen abgetrennt werden kann.

So gewonnener Wasserstoff eignet sich beispielsweise zur Synthese von Ammoniak, in Raffinerieprozessen zur Herstellung von Benzin und Krackprodukten der Petrochemie, zur Methanolsynthese, zur Fetthärtung u.a. Hydrierungen, als Reduktionsmittel zur Gewinnung von W, Mo, Co u.a. Metallen, als reduzierendes Schutzgas bei metallurgischen Prozessen, zu autogenen Schweißen und Schneiden, als Brenngas in Mischung mit anderen Gasen (Stadtgas, Wassergas), oder verflüssigt als Treibstoff in Luft- und Raumfahrt.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, ebenso als Zwischenprodukt zur Herstellung von Polymeren, wie Polyoxymethylen, Polyacetal, Phenolharzen, Melaminen, 1,4-Butanol, Trimethylolpropan, Neopentylglykol, Pentaerythrit und Trioxan, sowie für methanolische Formaldehydlösungen und Methylal.

Da Formaldehyd nach dem erfindungsgemäßen Verfahren üblicherweise mit geringem Wassergehalt hergestellt wird, eignet sich so hergestellter Formaldehyd insbesondere für de Polymerisation zu Polyoxymethylen und Trioxan, da hier wasserfreier Formaldehyd einzusetzen ist.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, ebenso als Zwischenprodukt zur Herstellung von Kunststoffen, beispielsweise Polyoxymethylenen, Polyacetalen, Phenolharzen, Melaminen, Aminoplasten, Polyurethanen und Caseinkunststoffen, sowie 1,4-Butanole, Trimethylpropan, Neopentylglykol, Pentaerythrit und Trioxan, zur Herstellung von Farbstoffen, wie Fuchsin, Acridin, zur Herstellung von Düngemitteln sowie zur Behandlung von Saatgut.

Da Formaldehyd nach dem erfindungsgemäßen Verfahren üblicherweise mit geringem Wassergehalt hergestellt wird, eignet sich so hergestellter Formaldehyd insbesondere für die Polymerisation von Polyoxymethylen und Trioxan, da hier wasserfreier Formaldehyd einzusetzen ist.

Die Erfindung betrifft auch derartig hergestellte Kunststoffe, wie Polyoxymethylen, und Polyacetale, Trioxan, Farbstoffe, Düngemittel und Saatgut.

Weiterhin Gegenstand der Erfindung ist ein Verfahren zur Herstellung von Trioxan, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man dem Reaktor einen Trägergasstrom zuführt, der eine Temperatur aufweist, die oberhalb der Dehydrierungstemperatur liegt, und
2. den so hergestellten Formaldehyd zu Trioxan trimerisiert.

Einzelheiten der Herstellung von Trioxan sind dem Fachmann bekannt und geläufig. Sie sind z.B. in Kirk u. Othmer, Encyclopedia of Chemical Technology, 2. Aufl., Band 10, S. 83, 89, New York Interscience 1963-1972, beschrieben.

Gegenstand der Erfindung ist ebenso ein Verfahren zur Herstellung von Polyoxymethylen, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man dem Reaktor einen Trägergasstrom zuführt, der eine Temperatur aufweist, die oberhalb der Dehydrierungstemperatur liegt, und
2. den so gewonnenen Formaldehyd gegebenenfalls reinigt,
3. den Formaldehyd polymerisiert,
4. die Endgruppen des so hergestellten Polymers absättigt (Capping) und
5. gegebenenfalls das Polymer in der Schmelze homogenisiert und/oder mit geeigneten Zusätzen versieht.

Die Herstellung von Polyoxymethylen aus Formaldehyd ist dem Fachmann bekannt und geläufig. Einzelheiten finden sich z.B. in Ullmann's Encyclopedia of Industrial Chemistry, Bd. 21, 5. Aufl., Weinheim 1992, und der dort zitierten Literatur.

Ein weiterer Gegenstand der Erfindung ist ein Verfahren zur Herstellung Polyoxymethylen Copolymeren, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man dem Reaktor einen Trägergasstrom zuführt, der eine Temperatur aufweist, die oberhalb der Dehydrierungstemperatur liegt, und
2. den so gewonnenen Formaldehyd zu Trioxan trimerisiert,
3. gegebenenfalls das Trioxan reinigt,
4. das Trioxan mit cyclischen Ethern oder cyclischen Acetalen copolymerisiert,
5. gegebenenfalls instabile Endgruppen entfernt und
6. das so hergestellte Polymer gegebenenfalls in der Schmelze homogenisiert und/oder mit geeigneten Zusatzstoffen versetzt.

Ein weiterer Gegenstand der Erfindung ist ein Verfahren zur Herstellung Polyoxymethylen Copolymeren, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
2. den so gewonnenen Formaldehyd gegebenenfalls reinigt,
3. den Formaldehyd mit cyclischen Ethern oder cyclischen Acetalen copolymerisiert,
4. gegebenenfalls instabile Endgruppen entfernt und

5. das so hergestellte Polymer gegebenenfalls in der Schmelze homogenisiert und/oder mit geeigneten Zusatzstoffen versetzt.

Die Herstellung von Polyoxymethylen-Copolymeren ist dem Fachmann bekannt und geläufig. Einzelheiten finden sich beispielsweise in Ullmann's Encyclopedia of Industrial Chemistry, Bd. 21, 5. Aufl., Weinheim 1992 und der dort zitierten Literatur, sowie in russischen Schriften SU 436067, 740715 und SU 72-1755156, 720303.
Auf den Inhalt der prioritätsbegründenden deutschen Patentanmeldungen 197 22 774.0, 197 27 519.2 und 19743145.3 sowie auf die Zusammenfassung der vorliegenden Anmeldung wird ausdrücklich Bezug genommen. Sie gelten durch Zitat als Bestandteil der Beschreibung.
Die Erfindung wird durch die Beispiele näher erläutert, ohne sie dadurch einzuschränken.

Beispiele

Figur 2 zeigt schematisch anhand eines Fließbildes den Aufbau der Versuchsapparatur.
Die Dehydrierung des Methanols wird in einem Rohrreaktor 26, der indirekt durch einen elektrischen Rohrofen 12 beheizt wird, durchgeführt. Eine Katalysatorzugabeeinheit wird durch ein Metallrohr 11 gebildet, das indirekt durch den elektrischen Rohrofen 12 beheizt wird. In dem Rohr 11 ist eine Schüttung 13 aus Trägermaterial angeordnet, auf der Primärkatalysator (0,1 - 5,0 g) vorgelegt ist. In dieses Rohr 11 wird ein Teil 14 eines überhitzten Trägergasstromes 15 geleitet, der vorher über beheizte Zuleitungen vorgewärmt wurde. In dieses Rohr 11 wird außerdem Primärkatalysator als Lösung über eine Düse 16 nachgeführt. Der Primärkatalysator schlägt sich auf der Schüttung 13 nieder.
Der Trägergasteilstrom 14 wird durch die Schüttung geleitet, um ihn mit einer sich bildenden aktiven Katalysator-Spezies zu beladen.
Der Gesamtstrom wird anschließend in den Reaktionsraum 19 geleitet.
Methanol 17 wird vorgewärmt und in einem weiteren Teil 18 des Trägergasstromes 15 gefördert und ebenfalls dem Reaktionsraum 19 zugegeben.
Ein dritter Gasstrom 20, der aus reinem Trägergas 15 besteht, wird überhitzt 21, d.h. auf eine Temperatur gebracht, die über der Dehydrierungstemperatur liegt, und ebenfalls in den Reaktionsraum 19 geleitet.
Der Reaktionsraum 19 wird durch ein Rohr mit der Länge 200-450 mm, Innendurchmesser 4-21 mm gebildet. Die Produktgase werden in einem Kühler 22 nach Austritt aus dem Reaktionsraum 19 rasch auf eine Temperatur kleiner 200 °C gekühlt und mittels eines Gaschromatographen analysiert.
In einer Kolonne 23 werden die Reaktionsprodukte mit Alkohol 24 (z.B. Cyclohexanol bei 20-80 °C) gewaschen, um den Formaldehyd 25 auszutragen.
Als Primärkatalysator wird Natriummethylat verwendet, als Trägergas $H_2/CO$ oder Stickstoff. Der Gesamtstrom beträgt 20-500 l/h, mindestens 50 % des Trägergasstromes werden nach der Überhitzung direkt in den Reaktor geführt. Die Methanolzufuhr ist so bemessen, daß sich eine Methanol-Konzentration von ca. 5-20 mol-% einstellt.
Die angegebenen Meßgrößen werden wie folgt berechnet:

$$\text{Ausbeute (in \%)} = \frac{\text{gebildeter Formaldehyd (mol)}}{\text{zudosiertes Methanol (mol)}} \cdot 100$$

| Beispiel/ Vergleichsbeispiel | Ofentemperatur Katalysatorzersetzung | Temperatur Trägergasstrom | Ofentemperatur Reaktor Dehydrierung | Ausbeute Formaldehyd |
|---|---|---|---|---|
| Bsp. 1 | 900°C | 870°C | 750°C | 76 % |
| Bsp. 2 | 880°C | 870°C | 750°C | 74 % |
| VB 1 | 900°C | 820°C | 750°C | 72 % |

**Patentansprüche**

1. Verfahren zur Herstellung von Formaldehyd aus Methanol durch Dehydrierung des Methanols bei einer Temperatur im Bereich von 300 bis 1000°C in Gegenwart einer aus einer Natriumverbindung freigesetzten katalytisch wirksamen Spezies, **dadurch gekennzeichnet, daß** man aus einer Natriumverbindung aus der Gruppe

   a) Natriumalkoholate
   b) Natriumcarboxylate

c) Natriumsalze C-H acider Verbindungen
d) Natriumoxid, Natriumhydroxid, Natriumnitrit, Natriumacetylid, Natriumcarbid, Natriumhydrid und Natriumcarbonyl

durch thermische Zersetzung die katalytisch aktive Spezies freisetzt.

**2.** Verfahren nach Anspruch 1, **dadurch gekennzeichnet, daß** man eine Natriumverbindung aus der Gruppe

a) Natriumalkoholate niederer ($C_1$ bis $C_4$) Alkohole
b) Natriumcarboxylate niederer ($C_1$ bis $C_4$) Mono-, Di-, Tri- und Hydroxycarbonsäuren
c) Na-Acetylacetonat, Na-Acetessigester
d) Natriumcarbid

einsetzt.

**3.** Verfahren nach Anspruch 1, **dadurch gekennzeichnet, daß** man eine Natriumverbindung aus der Gruppe

a) $NaOCH_3$
b) NaOOCH, $NaOOC\text{-}CH_3$, $NaOOC\text{-}CH(OH)\text{-}CH_3$
c) $Na_2C_2$

einsetzt.

**4.** Verfahren nach Anspruch 1, **dadurch gekennzeichnet, daß** man als Natriumverbindung $NaOCH_3$, NaOOCH, $NaOOC\text{-}CH_3$, und/oder $Na_2C_2$ einsetzt.

**5.** Verfahren nach einem oder mehreren der Ansprüche 1 bis 4 **dadurch gekennzeichnet, daß** die Natriumverbindung als Feststoff, in einem Lösungsmittel gelöst, als Flüssigkeit oder als Schmelze jeweils kontinuierlich oder diskontinuierlich zu- bzw. nachgeführt wird.

**6.** Verfahren nach einem oder mehreren der Ansprüche 1 bis 5, **dadurch gekennzeichnet, daß** die Dehydrierung in einem Reaktor durchgeführt wird und man die Erzeugung der katalytisch aktiven Spezies räumlich getrennt vom Reaktor durchführt.

**7.** Verfahren nach einem oder mehreren der Ansprüche 1 bis 6, **dadurch gekennzeichnet, daß** die Dehydrierung in einem Reaktor durchgeführt wird und man einen Kreisgasstrom, der im wesentlichen aus den Nebenprodukten der Dehydrierung besteht, durch den Reaktor führt.

**8.** Verfahren nach einem oder mehreren der Ansprüche 1 bis 7, **dadurch gekennzeichnet daß** die Dehydrierung in einem Reaktor durchgeführt wird und man einen Teil der Nebenprodukte der Dehydrierung als Brennstoff zur Beheizung des Reaktors verwendet.

**9.** Verfahren nach einem oder mehreren der Ansprüche 1 bis 8, **dadurch gekennzeichnet, daß** man das Methanol mit einem Trägergas verdünnt und den Trägergasstrom erwärmt, bevor er mit dem Methanol zusammengeführt wird.

**10.** Verfahren nach einem oder mehreren der Ansprüche 1 bis 5, **dadurch gekennzeichnet, daß** man den als Nebenprodukt anfallenden Wasserstoff abtrennt und einer weiteren Verwertung zuführt.

**11.** Verwendung von Natriumverbindungen gemäß einem oder mehreren der Ansprüche 1 bis 4 zur Erzeugung einer katalytisch aktiven Spezies in einem Verfahren zur Herstellung von Formaldehyd aus Methanol durch Dehydrierung des Methanols bei einer Temperatur im Bereich von 300°C bis 1000°C.

**12.** Verfahren zur Herstellung von Trioxan, **dadurch gekennzeichnet, daß** man Methanol durch Dehydrierung bei einer Temperatur im Bereich von 300°C bis 1000°C in Gegenwart einer aus einer Natriumverbindung freigesetzten katalytisch wirksamen Spezies zu Formaldehyd umsetzt, wobei man eine Natriumverbindung aus der Gruppe

a) Natriumalkoholate

b) Natriumcarboxylate
c) Natriumsalze C-H acider Verbindungen
d) Natriumoxid, Natriumhydroxid, Natriumnitrit, Natriumacetylid, Natriumcarbid, Natriumhydrid und Natriumcarbonyl

einsetzt, und den so hergestellten Formaldehyd zu Trioxan trimerisiert.

**13.** Verfahren zur Herstellung von Polyoxymethylen, **dadurch gekennzeichnet, daß** man Methanol durch Dehydrierung bei einer Temperatur im Bereich von 300°C bis 1000°C in Gegenwart einer aus einer Natriumverbindung freigesetzten katalytisch wirksamen Spezies zu Formaldehyd umsetzt, wobei man eine Natriumverbindung aus der Gruppe

a) Natriumalkoholate
b) Natriumcarboxylate
c) Natriumsalze C-H acider Verbindungen
d) Natriumoxid, Natriumhydroxid, Natriumnitrit, Natriumacetylid, Natriumcarbid, Natriumhydrid und Natriumcarbonyl

einsetzt, und

- den so hergestellten Formaldehyd gegebenenfalls reinigt,
- den Formaldehyd polymerisiert,
- die Endgruppen des so hergestellten Polymers absättigt (Capping) und
- gegebenenfalls das Polymer in der Schmelze homogenisiert und/oder mit geeigneten Zusätzen versieht .

**14.** Verfahren zur Herstellung von Polyoxymethylen-Copolymeren, **dadurch gekennzeichnet, daß** man Methanol durch Dehydrierung bei einer Temperatur im Bereich von 300°C bis 1000°C in Gegenwart einer aus einer Natriumverbindung freigesetzten katalytisch wirksamen Spezies zu Formaldehyd umsetzt, wobei man eine Natriumverbindung aus der Gruppe

a) Natriumalkoholate
b) Natriumcarboxylate
c) Natriumsalze C-H acider Verbindungen
d) Natriumoxid, Natriumhydroxid, Natriumnitrit, Natriumacetylid, Natriumcarbid, Natriumhydrid und Natriumcarbonyl

einsetzt, und

- den so hergestellten Formaldehyd zu Trioxan trimerisiert,
- gegebenenfalls das Trioxan reinigt,
- das Trioxan mit cyclischen Ethem oder cyclischen Acetalen copolymerisiert
- gegebenenfalls instabile Endgruppen entfernt und
- das so hergestellte Polymer gegebenenfalls in der Schmelze homogenisiert und/oder mit geeigneten Zusätzen versieht.

**15.** Verfahren zur Herstellung von Polyoxymethylen-Copolymeren, **dadurch gekennzeichnet, daß** man Methanol durch Dehydrierung bei einer Temperatur im Bereich von 300°C bis 1000°C in Gegenwart einer aus einer Natriumverbindung freigesetzten katalytisch aktiven Spezies zu Formaldehyd umsetzt, wobei man eine Natriumverbindung aus der Gruppe

a) Natriumalkoholate
b) Natriumcarboxylate
c) Natriumsalze C-H acider Verbindungen
d) Natriumoxid, Natriumhydroxid, Natriumnitrit, Natriumacetylid, Natriumcarbid, Natriumhydrid und Natriumcarbonyl

einsetzt, und

- den so hergestellten Formaldehyd gegebenenfalls reinigt,
- den Formaldehyd mit cyclischen Ethern oder cyclischen Acetalen copolymerisiert,
- gegebenenfalls instabile Endgruppen entfernt und
- das so hergestellte Polymer gegebenenfalls in der Schmelze homogenisiert und/oder mit geeigneten Zusätzen versieht.

Fig. 1

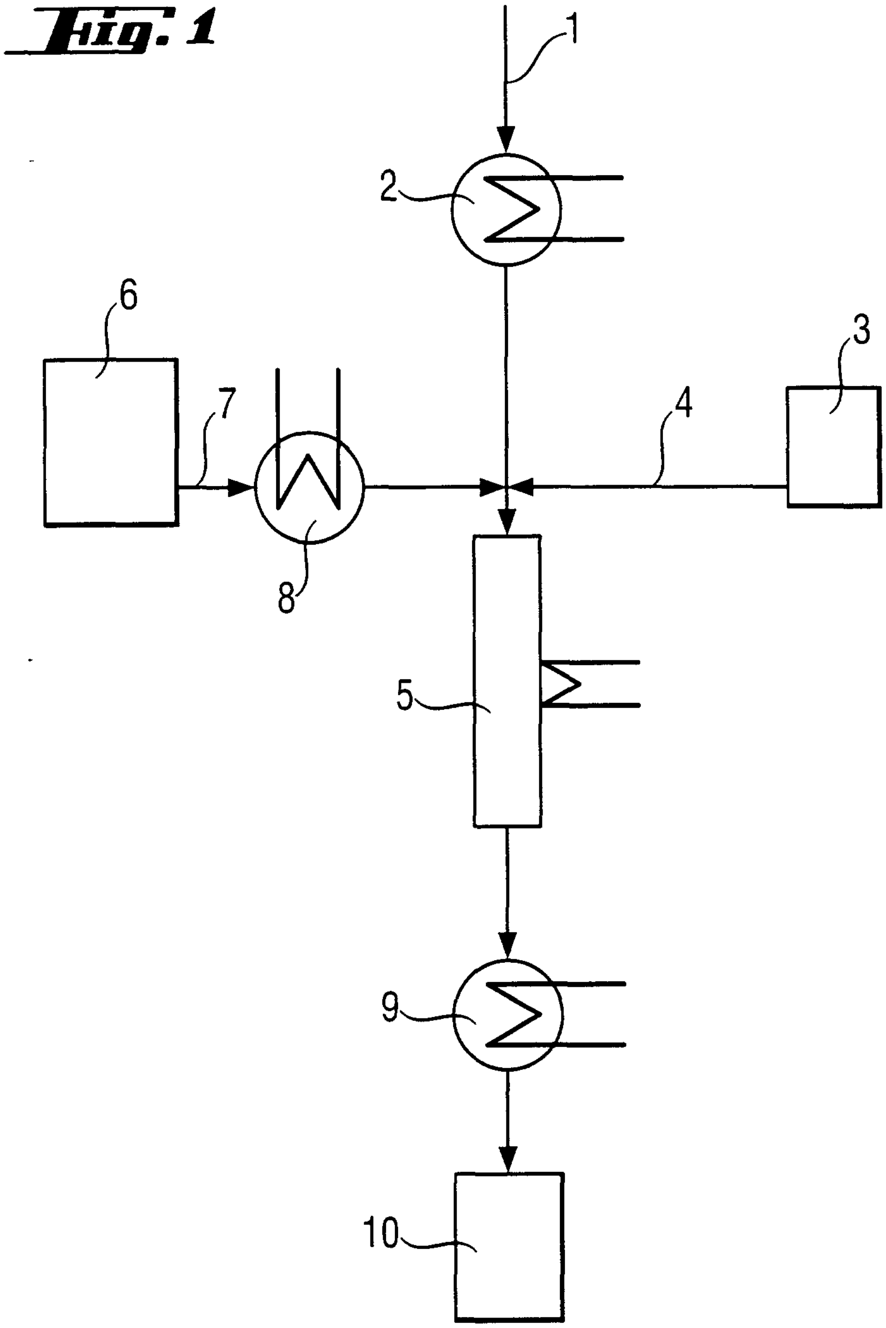

Fig. 2

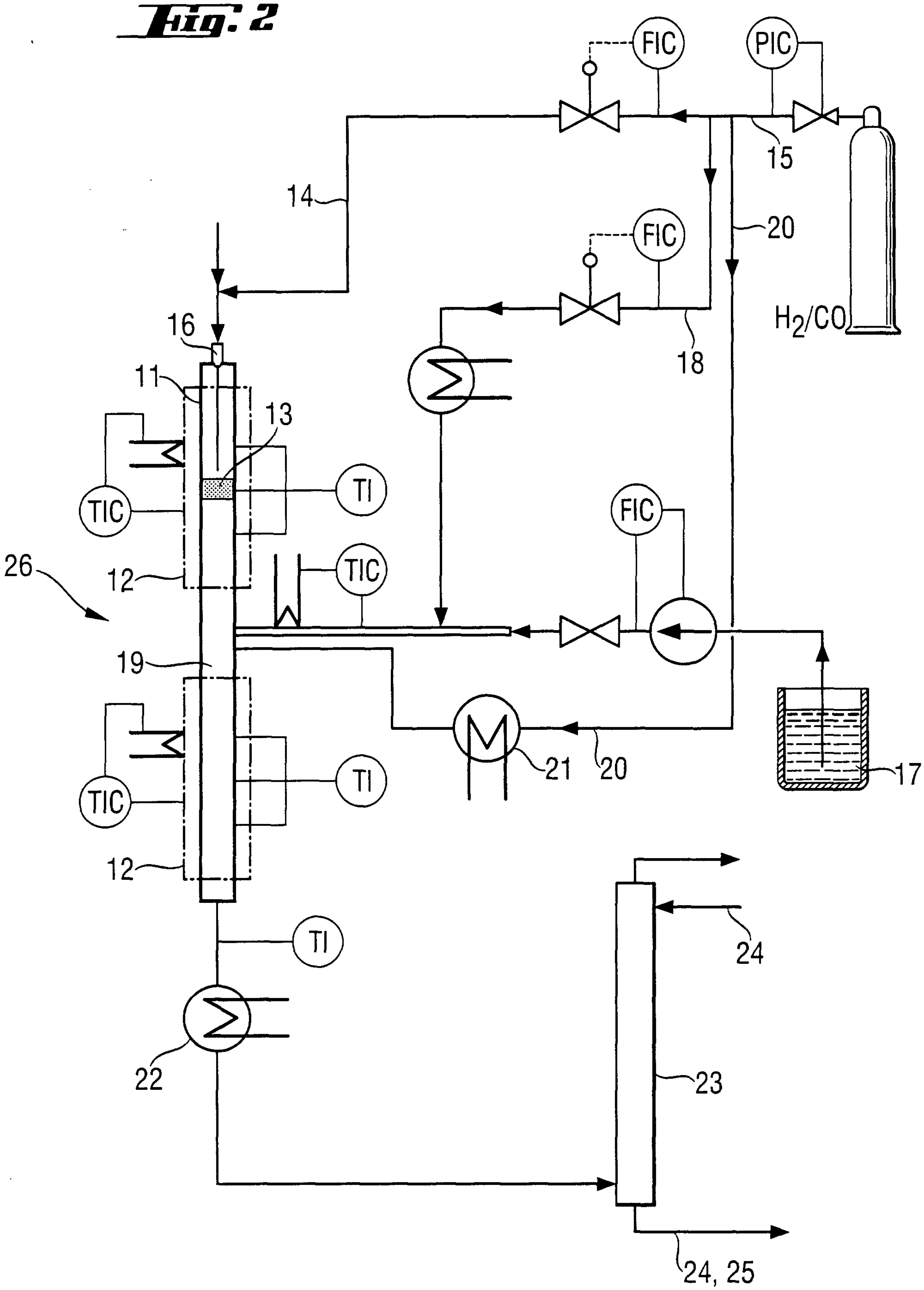
FIC
PIC
15
14
FIC
20
16
H2/CO
18
11
13
TI
TIC
TIC
26
12
FIC
19
TI
TIC
21
20
17
12
TI
24
22
23
24, 25

Europäisches Patentamt

# EUROPÄISCHER RECHERCHENBERICHT

Nummer der Anmeldung
EP 01 11 8017

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| D,X | J. SAUER ET AL.: "The Catalyzed Dehydrogenation of Methanol to Formaldehyd at High Temperatures" CHEM. ENG TECHNOL., Bd. 18, 1995, Seiten 284--291, XP002077501 * Absatz '0005! - Absatz '0007! * | 1-15 | C07C45/00<br>C07C47/04<br>B01J23/04 |
| D,X | DE 37 19 055 A (HOECHST AG) 15. Dezember 1988 (1988-12-15) * Spalte 2, Zeile 19 - Zeile 21; Anspruch 1 * | 1-15 | |
| | | | RECHERCHIERTE SACHGEBIETE (Int.Cl.7) |
| | | | C07C<br>B01J |

Der vorliegende Recherchenbericht wurde für alle Patentansprüche erstellt

| Recherchenort | Abschlußdatum der Recherche | Prüfer |
|---|---|---|
| MÜNCHEN | 17. September 2001 | Janus, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 01 11 8017

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-09-2001

| Im Recherchenbericht angeführtes Patentdokument | Datum der Veröffentlichung | Mitglied(er) der Patentfamilie | Datum der Veröffentlichung |
|---|---|---|---|
| DE 3719055 A | 15-12-1988 | EP 0294684 A | 14-12-1988 |

EPO FORM P0461

Für nähere Einzelheiten zu diesem Anhang : siehe Amtsblatt des Europäischen Patentamts, Nr.12/82